(19) Europäisches Patentamt
European Patent Office
Office européen des brevets

(11) Numéro de publication : **0 423 010 B1**

(12) **FASCICULE DE BREVET EUROPEEN**

(45) Date de publication du fascicule du brevet : **29.09.93 Bulletin 93/39**

(51) Int. Cl.$^5$ : **C07C 45/64,** C07C 47/575, C07C 47/58

(21) Numéro de dépôt : **90402786.9**

(22) Date de dépôt : **08.10.90**

(54) **Procédé de préparation d'alcoxybenzaldéhydes.**

(30) Priorité : **09.10.89 FR 8913370**

(43) Date de publication de la demande : **17.04.91 Bulletin 91/16**

(45) Mention de la délivrance du brevet : **29.09.93 Bulletin 93/39**

(84) Etats contractants désignés : **AT BE CH DE DK ES FR GB GR IT LI LU NL SE**

(56) Documents cités :
**EP-A- 0 155 335**
**DE-B- 2 248 337**
**DE-C- 723 412**
**GB-A- 2 089 672**
**US-A- 4 218 567**

(56) Documents cités :
**Canadian Journal of Chemistry, vol. 31, no. 5, mai 1953, National Research Council, Ottawa, CA; pages 476 - 483; J. M. Pepper: "The synthesis of syringaldehyde from vanillin"**

(73) Titulaire : **RHONE-POULENC CHIMIE**
**25, quai Paul Doumer**
**F-92408 Courbevoie Cédex (FR)**

(72) Inventeur : **Nobel, Dominique**
**7, rue de Saint Cloud**
**F-69007 Lyon (FR)**

(74) Mandataire : **Dutruc-Rosset, Marie-Claude et al**
**RHONE-POULENC CHIMIE Service Brevets Chimie 25, Quai Paul Doumer**
**F-92408 Courbevoie Cédex (FR)**

EP 0 423 010 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet un procédé de préparation d'alcoxybenzaldéhydes à partir d'halogénobenzaldéhydes. L'invention vise plus particulièrement la préparation d'alcoxy-3 hydroxy-4 benzaldéhydes à partir d'halogéno-3 hydroxy-4 benzaldéhydes et d'alcoxy-5 hydroxy-2 benzaldéhydes à partir d'halogéno-5 hydroxy-2 benzaldéhydes.

Un article de Canadian Journal of Chemistry 31, page 476 (1953) décrit la méthoxylation des halogéno-5 hydroxy-4 méthoxy-3 benzaldéhydes (ou halogéno-5 vanillines) par le méthylate de sodium, dans le méthanol et en présence d'un composé du cuivre II.

Le rendement obtenu avec le dérivé bromé est d'environ 60 %.

Le brevet américain n°4218567 décrit notamment la préparation du diméthoxy-3,5 hydroxy-4 benzaldéhyde à partir du bromo-5 méthoxy-3 hydroxy-4 benzaldéhyde, par réaction avec un alcoolate alcalin en présence du cholure cuivreux dans la diméthylformamide.

Le brevet GB-A-2089672 décrit la préparation de diméthoxy-3,5 hydroxy-4 benzaldéhyde à partir du bromo-5 hydroxy-4 méthoxy-3 benzaldéhyde et du méthylate de sodium, en présence d'un catalyseur constitué d'un ester de l'acide formique et d'un sel de cuivre I. Dans ce procédé la fonction aldéhyde est préalablement protégée en le transformant en acétal. La réaction est très lente.

La présente invention se propose de réaliser l'alkoxylation des halogénobenzaldéhydes dans un solvant, de préférence, un alcool et sans qu'il soit nécessaire de protéger la fonction aldéhyde sous forme d'acétal.

Dans l'exposé qui suit de la présente invention, on entend par "halogénobenzaldéhyde", tout composé aromatique portant au moins une fonction aldéhyde et au moins un atome d'halogène.

Plus précisément, l'invention consiste en un procédé de préparation d'alcoxybenzaldéhydes par réaction :

- d'un halogénobenzaldéhyde de formule générale (I) :

$$X - R_o - CHO \qquad (I)$$

  dans laquelle :
  - X représente un atome d'halogène, de préférence un atome d'iode, de brome ou de chlore,
  - $R_o$ représente un radical cyclique aromatique ayant au moins 5 atomes dans le cycle, éventuellement substitué, et représentant au moins l'un des radicaux suivants :
    . un radical carbocyclique aromatique, monocyclique ou polycyclique,
    . un radical hétérocyclique aromatique, monocyclique ou polycyclique comportant au moins un des hétéroatomes O, N et S,
- et d'un alcoolate de métal alcalin de formule générale (II) :

$$M^{m+} [ O - R ]_m^{-} \qquad (II)$$

  dans laquelle :
  - M représente un métal alcalin ou alcalino-terreux,
  - m représente la valence du métal alcalin ou alcalino-terreux,
  - R représente un radical hydrocarboné ayant de 1 à 12 atomes de carbone, qui peut être un radical aliphatique acyclique saturé ou insaturé, linéaire ou ramifié ou un radical arylaliphatique

en présence de cuivre et/ou d'un composé du cuivre caractérisé par le fait que l'on opère en présence d'une quantité efficace d'un carbonate organique, d'un carbonate mixte organo-métallique, de dioxyde de carbone ou d'un composé susceptible de former du dioxyde de carbone dans le milieu réactionnel.

L'invention s'applique plus particulièrement aux composés de formule (I) dans laquelle le radical $R_o$ symbolise :

1° - un radical carbocyclique aromatique, monocyclique ou polycyclique.

Par "radical carbocyclique polycyclique", on entend :

. un radical constitué par au moins 2 carbocycles aromatiques et formant entre eux des systèmes ortho ou ortho et péricondensés,

. un radical constitué par au moins 2 carbocycles dont l'un seul d'entre eux est aromatique et formant entre eux des systèmes ortho ou ortho et péricondensés.

2° - un radical hétérocyclique aromatique, monocyclique ou polycyclique.

Par "radical hétérocyclique polycyclique", on définit :

. un radical constitué par au moins 2 hétérocycliques contenant au moins un hétéroatome dans chaque cycle dont au moins l'un des deux cycles est aromatique et formant entre eux des systèmes ortho ou ortho et péricondensés,

. un radical constitué par au moins un cycle hydrocarboné et au moins un hétérocycle dont au moins l'un des cycles est aromatique et formant entre eux des systèmes ortho ou ortho et péricondensés.

3° - un radical divalent constitué par un enchaînement de groupements, tels que définis aux paragraphes 1 et/ou 2 liés entre eux :

. par un lien valentiel,

. par un radical alkylène ou alkylidène ayant de 1 à 4 atomes de carbone, de préférence un radical méthylène ou isopropylidène,

. par l'un des groupes suivants :

$-O-$ , $-CO-$ , $-COO-$ ,

$-S-$ , $-SO-$ , $-SO_2-$ ,

−N− , −CO−N−
|      |
R'     R'

dans ces formules, R' représentant un radical alkyle ayant de 1 à 4 atomes de carbone, un radical cyclohexyle ou phényle.

Comme exemples de radicaux listés sous 1° à 3°, on peut citer :

1° - les radicaux phénylène, tolylène, xylylène, naphtylène,

2° - les radicaux furannediyle, pyrrolediyle, thiofènediyle, isoxazolediyle, furazannediyle, isothiazolediyle, imidazolediyle, pyrazolediyle, pyridinediyle, pyridazinediyle, pyrimidinediyle ; les radicaux naphtyridine-1,8 diyle, quinoléïnediyle, indolediyle, benzofurannediyle.

3° - les radicaux biphénylène, méthylène- 1,1' biphénylène, isopropylidène-1,1' biphénylène, oxy-1,1' biphénylène, imino-1,1' biphénylène.

Comme mentionné précédemment, le radical $R_o$ qui est un composé aromatique porteur d'au moins une fonction aldéhyde et d'au moins un atome d'halogène, peut être également porteur d'un autre substituant qui peut être un autre atome d'halogène ou de toute autre nature dans la mesure où il n'entre pas en compétition dans la réaction.

Comme exemples de substituants donnés à titre illustratif et sans caractère limitatif, on peut mentionner :

- un ou plusieurs radicaux alkyle ayant de 1 à 4 atomes de carbone,
- un ou plusieurs radicaux alcoxy ayant de 1 à 4 atomes de carbone,
- un ou plusieurs radicaux hydroxyle.

A titre d'exemples d'halogénobenzaldéhydes répondant à la formule générale (I), on peut citer plus particulièrement :

- le bromo-2 benzaldéhyde
- le bromo-3 benzaldéhyde
- le bromo-4 benzaldéhyde
- le bromo-5 méthoxy-2 benzaldéhyde
- le bromo-4 thiophènecarboxaldéhyde-2
- le bromo-5 thiophènecarboxaldéhyde- 3

Parmi les composés répondant à la formule générale (I), l'invention s'applique tout particulièrement aux halogénohydroxybenzaldéhydes répondant à la formule générale :

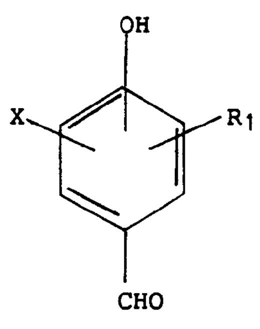

(Ia)

dans laquelle :

- X représente un atome d'iode, de brome ou de chlore,

- $R_1$ représente un atome d'hydrogène, un atome d'iode, de brome, un atome de chlore, un radical alkyle ayant de 1 à 4 atomes de carbone, un radical alcoxy ayant de 1 à 4 atomes de carbone, un radical hydroxyle,

dans ladite formule (Ia), le radical hydroxyle pouvant être en position ortho-, méta- ou para- de la fonction aldéhyde.

On peut citer plus particulièrement les composés répondant à la formule générale (Ia) dans laquelle :

. Groupe I :
  - le radical OH est en position para par rapport à la fonction CHO,
  - l'atome d'halogène X est en position ortho par rapport au radical OH,
  - le radical $R_1$ est en position ortho par rapport au radical OH.

. Groupe II :
  - le radical OH est en position ortho par rapport à la fonction CHO,
  - l'atome d'halogène X est en position para par rapport au radical OH,
  - le radical $R_1$ est en position ortho par rapport au radical OH.

. Groupe III :
  - le radical OH est en position ortho par rapport à la fonction CHO,
  - l'atome d'halogène X est en position ortho par rapport au radical OH,
  - le radical $R_1$ est en position para par rapport au radical OH,

. Groupe IV :
  - le radical OH est en position méta par rapport à la fonction CHO,
  - l'atome d'halogène X est en position ortho par rapport au radical OH,
  - le radical $R_1$ est en position para par rapport au radical OH.

. Groupe V :
  - le radical OH est en position méta par rapport à la fonction CHO,
  - l'atome d'halogène X est en position para par rapport au radical OH,
  - le radical $R_1$ est en position ortho par rapport au radical OH.

Les composés de formule (Ia) mis en oeuvre préférentiellement, répondent plus précisément aux formules suivantes :

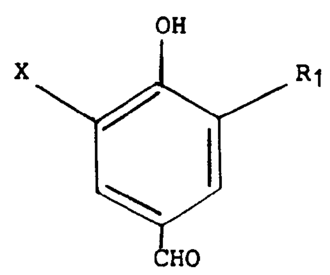

($Ia_1$)

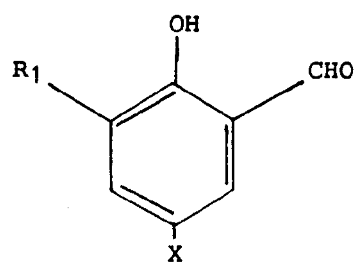

($Ia_2$)

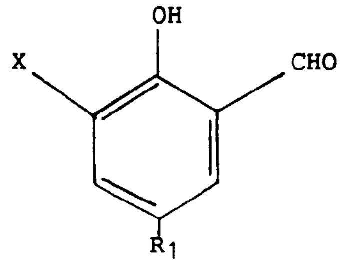

(Ia3)

dans lesquelles :

- X et $R_1$ ont les significations données précédemment.

A titre d'exemples d'halogénobenzaldéhydes répondant à la formule (Ia) qui servent de substrats de départ dans le présent procédé, on peut citer plus précisément :

- le bromo-3 hydroxy-4 benzaldéhyde,
- le iodo-3 hydroxy-4 benzaldéhyde
- le dibromo-3,5 hydroxy-4 benzaldéhyde
- le diiodo-3,5 hydroxy-4 benzaldéhyde
- le bromo-3 méthoxy-5 hydroxy-4 benzaldéhyde
- le iodo-3 méthoxy-5 hydroxy-4 benzaldéhyde
- le bromo-3 éthoxy-5 hydroxy-4 benzaldéhyde
- le iodo-3 éthoxy-5 hydroxy-4 benzaldéhyde
- le bromo-3 dihydroxy-4,5 benzaldéhyde
- le iodo-3 dihydroxy-4,5 benzaldéhyde
- le bromo-3 dihydroxy-2,5 benzaldéhyde
- le iodo-3 dihydroxy-2,5 benzaldéhyde
- le bromo-2 hydroxy-4 benzaldéhyde
- le iodo-2 hydroxy-4 benzaldéhyde
- le bromo-4 hydroxy-3 benzaldéhyde
- le iodo-4 hydroxy-3 benzaldéhyde
- le bromo-3 hydroxy-2 benzaldéhyde
- le iodo-3 hydroxy-2 benzaldéhyde
- le bromo-5 hydroxy-2 benzaldéhyde
- le iodo-5 hydroxy-2 benzaldéhyde

En ce qui concerne la définition de l'alcoolate de métal alcalin ou alcalino-terreux, on met en oeuvre tout particulièrement ceux répondant à la formule (II) dans laquelle R représente un radical alkyle, alcényle, alcadiényle, alcynyle linéaire ou ramifié ayant de préférence moins de 6 atomes de carbone ou un radical benzyle.

Parmi les alcoolates précités, on met en oeuvre de préférence dans le procédé de l'invention, les alcoolates de métaux alcalins et plus particulièrement les alcoolates de sodium ou de potassium des alcanols primaires ou secondaires ayant 1 à 4 atomes de carbone conviennent particulièrement bien.

Les plus fréquemment utilisés sont le méthylate de sodium et l'éthylate de sodium.

Conformément au procédé de l'invention, on fait réagir un halogénobenzaldéhyde de formule (I) avec un alcoolate de métal alcalin ou alcalino-terreux de formule (II) en présence d'un catalyseur au cuivre et d'une quantité efficace d'un carbonate organique, d'un carbonate mixte organo-métallique, de dioxyde de carbone ou d'un composé susceptible de former du dioxyde de carbone dans le milieu réactionnel.

Les carbonates organiques et les carbonates mixtes organo-métalliques utilisés dans l'invention sont plus particulièrement les carbonates de formule générale (III) :

$$[R_2\text{-O-CO-O-}]_n\text{-}R_3 \qquad \text{(III)}$$

dans laquelle :

- $R_2$ représente
  - un radical alkyle, linéaire ou ramifié, ayant 1 à 6 atomes de carbone ;
  - un radical aryle ;
  - un radical aryle substitué par 1 ou 2 radicaux alkyle ayant 1 à 12 atomes de carbone ;
  - un radical cycloalkyle ayant 5 à 6 atomes de carbone ;
  - un radical cycloalkyle, ayant 5 ou 6 atomes de carbone substitué par un ou deux radicaux alkyle ayant 1 à 12 atomes de carbone ;
  - un radical $R_4$-O-CO- dans lequel $R_4$ représente un radical alkyle, linéaire ou ramifié ayant 1 à 6 atomes de carbone, un radical aryle, un radical cycloalkyle ayant 5 ou 6 atomes de carbone ;

- $R_3$ représente
  - un radical alkyle, linéaire ou ramifié, ayant 1 à 6 atomes de carbone ;
  - un radical aryle ;
  - un radical aryle substitué par 1 ou 2 radicaux alkyle ayant 1 à 12 atomes de carbone ;
  - un radical cycloalkyle ayant 5 ou 6 atomes de carbone ;
  - un radical cycloalkyle ayant 5 ou 6 atomes de carbone substitué par un ou deux radicaux alkyle ayant 1 à 12 atomes de carbone;
  - un métal alcalin ou alcalino-terreux, de préférence le sodium ou le potassium ;
- n = 1 ou n = 2 lorsque $R_3$ représente un métal alcalino-terreux;
- $R_2$ et $R_3$ forment ensemble un radical alkylène ayant 2 à 6 atomes de carbone.

De préférence, les carbonates organiques et les carbonates mixtes organo-métalliques sont les carbonates de formule générale (IV) :

$$R_2\text{-O-CO-O-}R_3 \qquad (IV)$$

dans laquelle :

- $R_2$ représente
  - un radical alkyle, linéaire ou ramifié, ayant 1 à 6 atomes de carbone ;
  - un radical phényle ;
  - un radical phényle substitué par 1 ou 2 radicaux alkyle ayant 1 à 4 atomes de carbone ;
  - un radical cycloalkyle ayant 5 à 6 atomes de carbone ;
  - un radical cycloalkyle, ayant 5 ou 6 atomes de carbone substitué par un ou deux radicaux alkyle ayant 1 à 4 atomes de carbone ;
  - un radical $R_4$-O-CO- dans lequel $R_4$ représente un radical alkyle, linéaire ou ramifié ayant 1 à 6 atomes de carbone, un radical phényle, un radical cycloalkyle ayant 5 ou 6 atomes de carbone ;
- $R_3$ représente
  - un radical alkyle, linéaire ou ramifié, ayant 1 à 6 atomes de carbone ;
  - un radical phényle ;
  - un radical phényle substitué par un ou deux radicaux alkyle ayant 1 à 4 atomes de carbone ;
  - un radical cycloalkyle ayant 5 ou 6 atomes de carbone ;
  - un radical cycloalkyle ayant 5 ou 6 atomes de carbone substitué par un ou deux radicaux alkyle ayant 1 à 4 atomes de carbone ;
  - un atome de sodium ou de potassium ;
- $R_2$ et $R_3$ forment ensemble un radical alkylène ayant 2 à 6 atomes de carbone.

Le dioxyde de carbone peut être mis en oeuvre en solution dans le milieu liquide réactionnel par circulation dans celui-ci ou à l'aide d'une pression superatmosphérique.

Lorsque l'on utilise une pression de dioxyde de carbone, elle est généralement de 0,1 à 5 MPa.

Comme exemples de carbonates organiques ou organo-métalliques, on peut citer : le carbonate de ditertiobutyle, le carbonate de diéthyle, le carbonate de diméthyle, le carbonate d'éthylène, le carbonate de propylène, le carbonate de phényle et de tertiobutyle, le carbonate de tertiobutyle et de sodium, le dicarbonate de ditertiobutyle.

Comme exemples de composés susceptibles de former du dioxyde de carbone dans le milieu réactionnel, on peut citer :

- les $\alpha$(ou $\beta$)cétoesters et les $\alpha$(ou $\beta$)cétoacides ;
- les carbonates d'amine, les urées et les carbodiimides ;
- les acides dicarboxyliques comme l'acide malonique, les acides $\alpha$-cyano- ou $\alpha$-nitro-carboxyliques :
- les acides carboxyliques $\beta\gamma$-insaturés.

Les composés du cuivre servant de catalyseurs sont connus. Ce sont d'une manière générale tous les composés organiques ou inorganiques du cuivre I ou du cuivre II.

Le cuivre métal peut être utilisé, mais son action est plus lente car il faut préalablement qu'il se transforme en partie en cuivre I ou cuivre II.

A titre non limitatif, on peut citer comme composé du cuivre le chlorure cuivreux, le chlorure cuivrique, le carbonate cuivreux, le carbonate de cuivre II basique, le nitrate cuivreux, le nitrate cuivrique, le sulfate cuivrique, l'acétate cuivrique, le trifluorométhylsulfonate cuivrique, l'hydroxyde cuivrique, le picolinate de cuivre II, le méthylate de cuivre I, le méthylate de cuivre II, le chélate de cuivre II de la 8-quinoléine, les composés de cuivre de formule $ClCuOCH_3$, $Cu_2(OCH_3)_2(acac)_2$.

Conformément au procédé de l'invention, l'alkoxylation de l'halogénobenzaldéhyde répondant à la formule (I) est conduite en milieu organique constitué, le plus souvent, par l'alcanol correspondant à l'alcoolate de métal alcalin ou alcalino-terreux utilisé.

On choisit, préférentiellement comme solvant, le méthanol ou l'éthanol.

La concentration du composé de formule (I) exprimée en poids dudit composé (I) par rapport au poids total composé (I) + solvant est généralement de 3 à 40 % et, de préférence, de 10 à 30 %.

La quantité d'alcoolate de métal alcalin ou alcalino-terreux utilisée est égale ou supérieure à la quantité stoechiométrique nécessaire, d'une part pour transformer le composé de formule (I) en phénate de métal alcalin ou alcalino-terreux (c'est-à-dire pour salifier le ou les groupements (OH) et d'autre part pour transformer le ou les atomes d'halogène en groupements alcoxy.

Généralement l'alcoolate de métal alcalin ou alcalino-terreux mis en oeuvre représentera de 1 fois à 4 fois la quantité stoechiométrique ainsi définie et de préférence de 1,5 à 3 fois.

De manière pratique, l'alcoolate de métal alcalin ou alcalino-terreux est formé in situ, en faisant réagir un excès d'alcanol avec le métal alcalin ou alcalino-terreux choisi.

La quantité de catalyseur au cuivre utilisée dans le procédé de l'invention peut varier très largement.

Habituellement, le rapport molaire catalyseur au cuivre/composé de formule (I) est de 1 à 50 % et, de préférence, de 2 % à 20 %.

La quantité de carbonate organique ou organo-métallique de formule (III) ou (IV) habituellement utilisée est telle que l'on ait un rapport molaire carbonate de formule (III) ou (IV)/catalyseur au cuivre de 1 à 50 et, de préférence, de 1 à 10.

La température de la réaction d'alkoxylation est généralement comprise entre 90°C et 220°C, et, de préférence de 100°C à 180°C.

La pression n'est pas un paramètre critique en soi, sauf pour dissoudre le dioxyde de carbone lorsque celuici est utilisé, mais pour atteindre les températures indiquées précédemment et ne pas perdre de solvant, le procédé est habituellement réalisé sous pression autogène.

Généralement, cette pression autogène du mélange réactionnel est inférieure ou égale à 5 MPa (50 bars).

La durée de la réaction d'alkoxylation peut varier largement entre 2 et 10 heures, de préférence entre 3 et 5 heures.

Le procédé de l'invention est parfaitement bien adapté à la préparation du diméthoxy-3,5 hydroxy-4 benzaldéhyde (intermédiaire pour la préparation du triméthoxy-3,4,5 benzaldéhyde), de l'hydroxy-4 méthoxy-3 benzaldéhyde (vanilline) et de l'hydroxy-2 méthoxy-5 benzaldéhyde.

Un des avantages particulièrement intéressant du procédé de l'invention est qu'il permet d'effectuer la réaction d'alkoxylation sans qu'il soit nécessaire de protéger la fonction aldéhyde.

Un autre avantage du procédé de l'invention est qu'il fait appel à un solvant courant, peu onéreux et facilement recyclable.

Les exemples qui suivent illustrent l'invention.

EXEMPLE 1

Dans un réacteur téfloné de 40 $cm^3$, muni d'un système de chauffage et d'une agitation, on charge :

- 2,22 g (9,62 mmol) de bromo-3-hydroxy-4-méthoxy-5 benzaldéhyde (BHMB)
- 25 g d'une solution méthanolique contenant 2 g (37 mmol) de méthylate de sodium
- 0,099 g (1 mmol) de chlorure cuivreux.

On fait buller dans cette suspension du dioxyde de carbone anhydre pendant 30 secondes, puis on chauffe 3 heures à 125°C sous agitation et sous pression autogène. On refroidit à température ambiante, on dilue à l'eau distillée, on ajuste le pH du mélange réactionnel à 4 à l'aide d'acide sulfurique. On filtre la partie insoluble.

On dose par chromatographie en phase liquide.

Taux de transformation du BHMB (TT %) : 100 %.
Rendement (RT %) en syringaldéhyde
(hydroxy-4 diméthoxy-3,5 benzaldéhyde) : 97,5 %.

EXEMPLE 2

On répète l'exemple 1, avec les mêmes charges, les mêmes conditions opératoires, mais en travaillant sous une atmosphère d'azote et en remplaçant le dioxyde de carbone par 4 mmoles de dicarbonate de ditertiobutyle.

Après traitement on obtient les résultats suivants :

TT % du BHMB : 100 %
RT % en syringaldéhyde : 96 %.

ESSAI COMPARATIF A

On répète l'exemple 2, avec les mêmes charges, les mêmes conditions opératoires, en remplaçant le dicarbonate de ditertiobutyle par 0,70 g (11 mmol) de formiate de méthyle.

On obtient les résultats suivants :

TT % du BHMB : 68 %
RT % en syringaldéhyde : 93 %.

EXEMPLE 3

On répète l'exemple 1, avec les mêmes charges, les mêmes conditions opératoires, en remplaçant le dioxyde de carbone par 0,440 g (5 mmol) carbonate d'éthylène, le chlorure cuivreux par 0,110 g de carbonate de cuivre (II) basique.

Après traitement on obtient les résultats suivants :

TT % du BHMB : 98 %
RT % en syringaldéhyde : 93 %.

EXEMPLE 4

On répète l'exemple 3, avec les mêmes charges, les mêmes réactifs, les mêmes conditions opératoires, en remplaçant le carbonate d'éthylène par 0,42 g (4 mmol) de carbonate de propylène et en effectuant la réaction à 150°C.

Après traitement on obtient les résultats suivants :

TT % du BHMB : 100 %
RT % en syringaldéhyde : 89 %.

EXEMPLE 5

On répète l'exemple 3, avec les mêmes charges, les mêmes conditions opératoires, en remplaçant le carbonate d'éthylène par 0,5 $cm^3$ (4 mmol) de diéthylcarbonate.

Après traitement on obtient les résultats suivants :

TT % du BHMB : 97 %
RT % en syringaldéhyde : 96 %.

EXEMPLE 6

Dans un réacteur téfloné de 40 $cm^3$, muni d'un système de chauffage et d'une agitation, on charge :

- 2,10 g d'hydroxy-4-bromo-3-benzaldéhyde (HBB) à 95 %
- 2,16 g de méthylate de sodium
- 0,110 g de carbonate de cuivre basique
- 25 ml de méthanol.

On fait buller dans cette solution du dioxyde de carbone anhydre pendant 30 secondes, puis on chauffe 5 heures à 125°C (sous pression autogène) sous agitation. On refroidit à température ambiante, on dilue à l'eau distillée, on ajuste le pH du mélange réactionnel à 4 à l'aide d'acide sulfurique. On filtre la partie insoluble.

On dose par chromatographie en phase liquide.

TT % du HBB : 100 %.
RT % en vanilline : 99 %.

EXEMPLE 7

Dans un réacteur téfloné de 400 $cm^3$, muni d'un système de chauffage et d'une agitation, on charge :

- 21,3 g de bromo-3-hydroxy-4-méthoxy-5 benzaldéhyde (BHMB) (pureté 98 %)
- 21,6 g de méthylate de sodium
- 1,10 g de carbonate de cuivre basique
- 4,40 g de carbonate d'éthylène
- 250 $cm^3$ de méthanol.

On chauffe 3 heures à 125°C (sous pression autogène) sous agitation. On refroidit à 40°C, on dilue à l'eau distillée, on ajuste le pH du mélange réactionnel à 4 à l'aide d'acide sulfurique concentré, on filtre puis on évapore les solvant sous vide. On extrait deux fois au toluène chaud, on filtre et l'on évapore le toluène sous vide.

On obtient 17,25 g de syringaldéhyde (pureté 95 %).

Rendement en syringaldéhyde : 91 %

| | |
|---|---|
| Analyses | : C, 59,13 % |
| | H, 5,60 % |
| Calculé | : C, 59,33 % |
| | H, 5,53 % |

EXEMPLE 8

On répète l'exemple 7, avec les mêmes charges, les mêmes conditions opératoires, en remplaçant le carbonate d'éthylène par 5 $cm^3$ (59 mmol) de diméthylcarbonate.

On chauffe 3 heures à 150°C.

Après traitement on obtient les résultats suivants :

| | |
|---|---|
| TT % du BHMB | : 98 % |
| RT % en syringaldéhyde | : 94 %. |

EXEMPLE 9

On répète l'exemple 7, avec les mêmes charges, les mêmes conditions opératoires, en remplaçant le diméthylcarbonate par 5 $cm^3$ (40 mmol) de diéthylcarbonate.

On chauffe 3 heures 30 minutes à 150°C.

Après traitement on obtient les résultats suivants :

| | |
|---|---|
| TT % du BHMB | : 99 % |
| RT % en syringaldéhyde | : 92 %. |

EXEMPLE 10

Dans un réacteur téfloné de 40 $cm^3$, muni d'un système de chauffage et d'une agitation, on charge :

- 2,79 g de dibromo-3,5-hydroxy-4-benzaldéhyde (DBHB)
- 0,406 g de trifluorométhylsulfonate de cuivre II
- 2,70 g de méthylate de sodium
- 35 $cm^3$ de méthanol.

On fait buller dans cette suspension du dioxyde de carbone anhydre pendant 30 secondes puis on chauffe 4 heures à 150°C (sous pression autogène) sous agitation. Puis on procède comme dans l'exemple 1.

On obtient les résultats suivants :

| | |
|---|---|
| TT % du DBHB | : 100 % |
| RT % en syringaldéhyde | : 87 %. |
| RT % en bromovanilline | : 9 %. |

EXEMPLE 11

Dans un réacteur téfloné de 40 $cm^3$, muni d'un système de chauffage et d'une agitation, on charge :

- 4,1 g (20 mmol) de bromo-5 hydroxy-2 benzaldéhyde (BHB),
- 0,221 g (1 mmol) de carbonate cuivrique $Cu(OH)_2CuCO_3$,
- 15 $cm^3$ de méthanol.

On additionne sous agitation 14,4 g d'une solution méthanolique contenant 4,32 g (80 mmol) de méthylate de sodium.

On fait buller dans cette solution du dioxyde de carbone anhydre pendant 30 secondes, puis on chauffe 4 heures à 125°C (sous pression autogène) sous agitation. On refroidit à température ambiante, on dilue à l'eau distillée, on ajuste le pH du mélange réactionnel à 4 à l'aide d'acide sulfurique. On filtre la partie insoluble.

On dose par chromatographie en phase liquide.

Taux de transformation du BHB (TT %) : 58 %

Rendement (RT %) en hydroxy-2 méthoxy-5 benzaldéhyde : 79 %

## Revendications

1. Procédé de préparation d'alcoxybenzaldéhydes par réaction :
   - d'un halogénobenzaldéhyde de formule générale (I) :

$$X - R_o - CHO \qquad (I)$$

dans laquelle :

   - X représente un atome d'halogène, de préférence un atome d'iode, de brome ou de chlore,
   - $R_o$ représente un radical cyclique aromatique ayant au moins 5 atomes dans le cycle, éventuellement substitué, et représentant au moins l'un des radicaux suivants :
     . un radical carbocyclique aromatique, monocyclique ou polycyclique,
     . un radical hétérocyclique aromatique, monocyclique ou polycyclique comportant au moins un des hétéroatomes O, N et S,
   - et d'un alcoolate de métal alcalin de formule générale (II) :

$$M^{m+} [ O - R ]_m^- \qquad (II)$$

dans laquelle :

   - M représente un métal alcalin ou alcalino-terreux,
   - m représente la valence du métal alcalin ou alcalino-terreux,
   - R représente un radical hydrocarboné ayant de 1 à 12 atomes de carbone, qui peut être un radical aliphatique acyclique saturé ou insaturé, linéaire ou ramifié ou un radical arylaliphatique

   en présence de cuivre et/ou d'un composé du cuivre caractérisé par le fait que l'on opère en présence d'une quantité efficace d'un carbonate organique, d'un carbonate mixte organo-métallique, de dioxyde de carbone ou d'un composé susceptible de former du dioxyde de carbone dans le milieu réactionnel.

2. Procédé selon la revendication 1 caractérisé par le fait que l'halogénobenzaldéhyde répond à la formule générale (I) dans laquelle le radical $R_o$ symbolise :
   1°- un radical carbocyclique aromatique, monocyclique ou polycyclique.
   2° - un radical hétérocyclique aromatique, monocyclique ou polycyclique.
   3° - un radical divalent constitué par un enchaînement de groupements, tels que définis aux paragraphes 1 et/ou 2 liés entre eux :
     . par un lien valentiel,
     . par un radical alkylène ou alkylidène ayant de 1 à 4 atomes de carbone, de préférence un radical méthylène ou isopropylidène,
     . par l'un des groupes suivants :

$-O-$ , $-CO-$ , $-COO-$ ,

$-S-$ , $-SO-$ , $-SO_2-$ ,

$-N-$ , $-CO-N-$
$\ \ |\qquad\qquad\ \ |$
$\ R'\qquad\qquad R'$

dans ces formules, R' représentant un radical alkyle ayant de 1 à 4 atomes de carbone, un radical cyclohexyle ou phényle.

3. Procédé selon l'une des revendications 1 et 2 caractérisé par le fait que l'halogénobenzaldéhyde répond à la formule générale (Ia) :

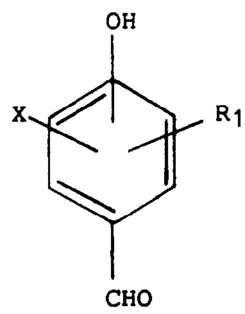

(Ia)

dans laquelle :

- X représente un atome d'iode, de brome ou de chlore,
- $R_1$ représente un atome d'hydrogène, un atome d'iode, de brome, un atome de chlore, un radical alkyle ayant de 1 à 4 atomes de carbone, un radical alcoxy ayant de 1 à 4 atomes de carbone, un radical hydroxyle,

dans ladite formule (Ia), le radical hydroxyle pouvant être en position ortho-, méta- ou para- de la fonction aldéhyde.

4. Procédé selon l'une des revendications 1 à 3 caractérisé par le fait que l'halogénobenzaldéhyde répond à la formule générale (Ia) dans laquelle :
   . <u>Groupe I</u> :
   - le radical OH est en position para par rapport à la fonction CHO,
   - l'atome d'halogène X est en position ortho par rapport au radical hydroxyle OH,
   - le radical $R_1$ est en position ortho par rapport au radical OH.

   . <u>Groupe II</u> :
   - le radical OH est en position ortho par rapport à la fonction CHO,
   - l'atome d'halogène X est en position para par rapport au radical OH,
   - le radical $R_1$ est en position ortho par rapport au radical OH.

   . <u>Groupe III</u> :
   - le radical OH est en position ortho par rapport à la fonction CHO,
   - l'atome d'halogène X est en position ortho par rapport au radical OH,
   - le radical $R_1$ est en position para par rapport au radical OH,

   . <u>Groupe IV</u> :
   - le radical OH est en position méta par rapport à la fonction CHO,
   - l'atome d'halogène X est en position ortho par rapport au radical OH,
   - le radical $R_1$ est en position para par rapport au radical OH.

   . <u>Groupe V</u> :
   - le radial OH est en position méta par rapport à la fonction CHO,
   - l'atome d'halogène X est en position para par rapport au radical OH,
   - le radical $R_1$ est en position ortho par rapport au radical OH.

5. Procédé selon l'une des revendications 1 à 4 caractérisé par le fait que l'halogénobenzaldéhyde répond à l'une des formules suivantes :

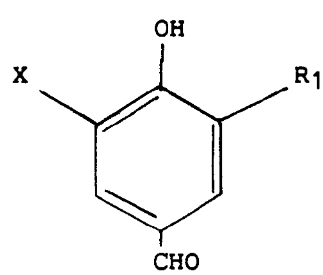

($Ia_1$)

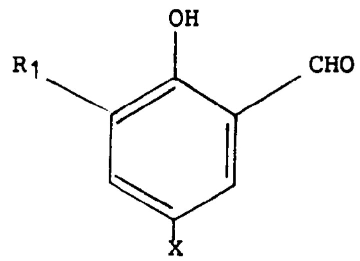

(Ia2)

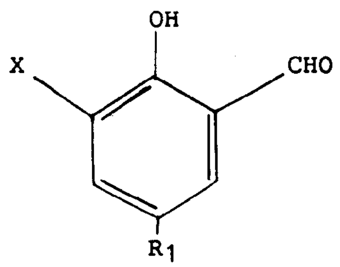

(Ia3)

dans lesquelles :

- X et $R_1$ ont les significations données précédemment.

6. Procédé selon l'une des revendications 1 à 5 caractérisé par le fait que l'halogénobenzaldéhyde est choisi parmi
   - le bromo-3 hydroxy-4 benzaldéhyde,
   - le iodo-3 hydroxy-4 benzaldéhyde
   - le dibromo-3,5 hydroxy-4 benzaldéhyde
   - le diiodo-3,5 hydroxy-4 benzaldéhyde
   - le bromo-3 méthoxy-5 hydroxy-4 benzaldéhyde
   - le iodo-3 méthoxy-5 hydroxy-4 benzaldéhyde
   - le bromo-3 éthoxy-5 hydroxy-4 benzaldéhyde
   - le iodo-3 éthoxy-5 hydroxy-4 benzaldéhyde
   - le bromo-3 dihydroxy-4,5 benzaldéhyde
   - le iodo-3 dihydroxy-4,5 benzaldéhyde
   - le bromo-3 dihydroxy-2,5 benzaldéhyde
   - le iodo-3 dihydroxy-2,5 benzaldéhyde
   - le bromo-2 hydroxy-4 benzaldéhyde
   - le iodo-2 hydroxy-4 benzaldéhyde
   - le bromo-4 hydroxy-3 benzaldéhyde
   - le iodo-4 hydroxy-3 benzaldéhyde
   - le bromo-3 hydroxy-2 benzaldéhyde
   - le iodo-3 hydroxy-2 benzaldéhyde
   - le bromo-5 hydroxy-2 benzaldéhyde
   - le iodo-5 hydroxy-2 benzaldéhyde

7. Procédé selon l'une des revendications 1 à 6 caractérisé par le fait que l'alcoolate de métal alcalin ou alcalino-terreux répond à la formule générale (II) dans laquelle R représente un radical alkyle, alcényle, alcadiényle, alcynyle linéaire ou ramifié ayant de préférence moins de 6 atomes de carbone ou un radical benzyle.

8. Procédé selon l'une des revendications 1 à 7 caractérisé par le fait que l'alcoolate de métal alcalin est un alcoolate de sodium ou de potassium d'un alcanol primaire ou secondaire ayant 1 à 4 atomes de carbone.

9. Procédé selon l'une des revendications 1 à 8 caractérisé par le fait que l'alcoolate de métal alcalin est le

méthylate de sodium ou l'éthylate de sodium.

**10.** Procédé selon l'une des revendications 1 à 9, caractérisé par le fait que l'on opère en présence d'un carbonate de formule générale (III) :

$$[R_2\text{-O-CO-O-}]_n\text{-}R_3 \qquad (III)$$

dans laquelle :
- $R_2$ représente
  - un radical alkyle, linéaire ou ramifié, ayant 1 à 6 atomes de carbone ;
  - un radical aryle ;
  - un radical aryle substitué par 1 ou 2 radicaux alkyle ayant 1 à 12 atomes de carbone ;
  - un radical cycloalkyle ayant 5 à 6 atomes de carbone ;
  - un radical cycloalkyle, ayant 5 ou 6 atomes de carbone substitué par un ou deux radicaux alkyle ayant 1 à 12 atomes de carbone ;
  - un radical $R_4$-O-CO- dans lequel $R_4$ représente un radical alkyle, linéaire ou ramifié ayant 1 à 6 atomes de carbone, un radical aryle, un radical cycloalkyle ayant 5 ou 6 atomes de carbone ;
- $R_3$ représente
  - un radical alkyle, linéaire ou ramifié, ayant 1 à 6 atomes de carbone ;
  - un radical aryle ;
  - un radical aryle substitué par 1 ou 2 radicaux alkyle ayant 1 à 12 atomes de carbone ;
  - un radical cycloalkyle ayant 5 ou 6 atomes de carbone ;
  - un radical cycloalkyle ayant 5 ou 6 atomes de carbone substitué par un ou deux radicaux alkyle ayant 1 à 12 atomes de carbone ;
  - un métal alcalin ou alcalino-terreux, de préférence le sodium ou le potassium ;
- n = 1 ou n = 2 lorsque $R_3$ représente un métal alcalino-terreux ;
- $R_2$ et $R_3$ forment ensemble un radical alkylène ayant 2 à 6 atomes de carbone.

**11.** Procédé selon l'une des revendications 1 à 10 caractérisé par le fait que l'on opère en présence d'un carbonate de formule générale (IV) :

$$R_2\text{-O-CO-O-}R_3 \qquad (IV)$$

dans laquelle :
- $R_2$ représente
  - un radical alkyle, linéaire ou ramifié, ayant 1 à 6 atomes de carbone ;
  - un radical phényle ;
  - un radical phényle substitué par 1 ou 2 radicaux alkyle ayant 1 à 4 atomes de carbone ;
  - un radical cycloalkyle ayant 5 à 6 atomes de carbone ;
  - un radical cycloalkyle, ayant 5 ou 6 atomes de carbone substitué par un ou deux radicaux alkyle ayant 1 à 4 atomes de carbone ;
  - un radical $R_4$-O-CO- dans lequel $R_4$ représente un radical alkyle, linéaire ou ramifié ayant 1 à 6 atomes de carbone, un radical phényle, un radical cycloalkyle ayant 5 ou 6 atomes de carbone ;
- $R_3$ représente
  - un radical alkyle, linéaire ou ramifié, ayant 1 à 6 atomes de carbone ;
  - un radical phényle ;
  - un radical phényle substitué par un ou deux radicaux alkyle ayant 1 à 4 atomes de carbone ;
  - un radical cycloalkyle ayant 5 ou 6 atomes de carbone ;
  - un radical cycloalkyle ayant 5 ou 6 atomes de carbone substitué par un ou deux radicaux alkyle ayant 1 à 4 atomes de carbone ;
  - un atome de sodium ou de potassium ;
- $R_2$ et $R_3$ forment ensemble un radical alkylène ayant 2 à 6 atomes de carbone.

**12.** Procédé selon l'une des revendications 1 à 11 caractérisé par le fait que l'on fait appel au dioxyde de carbone, au diméthylcarbonate, au diéthylcarbonate, au carbonate d'éthylène, de propylène, au dicarbonate de ditertiobutyle.

**13.** Procédé selon l'une des revendications 1 à 12, caractérisé par le fait que le catalyseur au cuivre est le cuivre métal ou un composé du cuivre qui est un composé organique ou inorganique du cuivre I ou du cuivre II, choisi parmi le chlorure cuivreux, le chlorure cuivrique, le carbonate cuivreux, le carbonate de cuivre II basique, le nitrate cuivreux, le nitrate cuivrique, le sulfate cuivrique, l'acétate cuivrique, le trifluorométhylsulfonate cuivrique, l'hydroxyde cuivrique, le picolinate de cuivre II, le méthylate de cuivre I, le

méthylate de cuivre II, le chélate de cuivre II de la 8-quinoléine, les composés du cuivre de formule $ClCuOCH_3$, $Cu_2(OCH_3)_2(acac)_2$.

**14.** Procédé selon l'une des revendications 1 à 13, caractérisé par le fait que la quantité d'alcoolate de métal alcalin ou alcalino-terreux utilisée est égale ou supérieure à la quantité stoechiométrique nécessaire pour transformer le composé de formule (I) en phénate alcalin ou alcalino-terreux correspondant et pour transformer le ou les atomes d'halogène qu'il comporte en groupements alcoxy.

**15.** Procédé selon l'une des revendications 1 à 14, caractérisé par le fait que le rapport molaire catalyseur au cuivre/composé de formule (I) est de 1 % à 50 % et de préférence de 2 % à 20 %.

**16.** Procédé selon l'une des revendications 1 à 15, caractérisé par le fait que la réaction est conduite dans un solvant constitué par l'alcanol correspondant à l'alcoolate de métal alcalin ou alcalino-terreux utilisé.

**17.** Procédé selon la revendication 16, caractérisé par le fait que la concentration initiale de composé de formule (I) par rapport au mélange composé de formule (I)/solvant est de 3 % à 40 % en poids par poids et de préférence de 10 % à 30 %.

**18.** Procédé selon l'une des revendications 1 à 17, caractérisé par le fait que le rapport molaire carbonate de formule (III) ou (IV)/catalyseur au cuivre est de 1 à 50 et de préférence de 1 à 10.

**19.** Procédé selon l'une des revendications 1 à 18, caractérisé par le fait que l'on opère à une température de 90°C à 220°C et de préférence de 100°C à 180°C.

**20.** Application du procédé décrit dans l'une des revendications 1 à 19 à la préparation du triméthoxy-3,4,5 benzaldéhyde, de l'hydroxy-4 méthoxy-3 benzaldéhyde et de l'hydroxy-2 méthoxy-5 benzaldéhyde.

## Claims

**1.** Process for the preparation of alkoxybenzaldehydes by the reaction of a halogenobenzaldehyde of general formula (I):

$$X\text{-}R_o\text{-}CHO \qquad (I)$$

in which

- X represents a halogen atom, preferably an iodine, bromine or chlorine atom.

$R_o$ represents an aromatic cyclic radical having at least 5 atoms in the cycle, which is optionally substituted and representing at least one of the fol lowing radicals:

- a monocyclic or polycyclic, aromatic carbocyclic radical,
- a monocyclic or polycyclic, aromatic heterocyclic radical having at least one of the heteroatoms O, N and S,
- and an alkali metal alkoxide of general formula (II):

$$M^{m+} [ O - R ]_{m-} \qquad (II)$$

in which:

- M represents an alkaline earth or alkali metal,
- m represents the valency of the alkaline earth or alkali metal,
- R represents a hydrocarbon radical having 1 to 12 carbon atoms, which can be a straight or branched, saturated or unsaturated, acyclic aliphatic radical or an aryl aliphatic radical

in the presence of copper and/or a copper compound, characterized in that working takes place in the presence of an effective quantity of an organic carbonate, mixed organometallic carbonate, carbon dioxide or a compound able to form carbon dioxide in the reaction medium.

**2.** Process according to claim 1, characterized in that the halogenobenzaldehyde is in accordance with the general formula (I), in which the radical $R_o$ symbolizes:

1° a monocyclic or polycyclic, aromatic carbocyclic radical,

2° a monocyclic or polycyclic, aromatic heterocyclic radical,

3° a divalent radical constituted by a chain of groups, such as defined in paragraphs 1 and/or 2 connected together:

- by a valency bond,
- by an alkylene or alkylidene radical having 1 to 4 carbon atoms, preferably a methylene or isopropylidene radical,
- by one of the following groups:

$-O-$, $-CO-$, $-COO-$,
$-S-$, $-SO-$, $-SO_2-$,

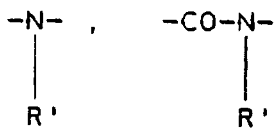

whereby in the said formulas R' represents an alkyl radical having 1 to 4 carbon atoms, a cyclohexyl or phenyl radical.

3. Process according to either of the claims 1 and 2, characterized in that the halogenobenzaldehyde complies with the general formula (Ia):

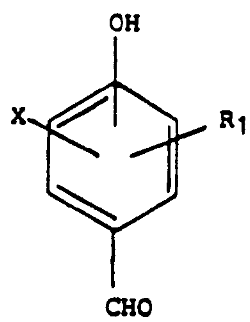

(Ia)

in which:
- X represents an iodine, bromine or chlorine atom,
- $R_1$ represents a hydrogen atom, an iodine, bromine or chlorine atom, an alkyl radical having 1 to 4 carbon atoms, an alkoxy radical having 1 to 4 carbon atoms, a hydroxyl radical,

whereby in said formula (Ia), the hydroxyl radical can be in the ortho, meta or para position of the aldehyde function.

4. Process according to any one of the claims 1 to 3, characterized in that the halogenobenzaldehyde complies with the general formula (Ia), in which:

GROUP I:
- the radical OH is in the para position relative to the CHO function,
- the halogen atom X is in the ortho position relative to the OH radical,
- the radical $R_1$ is in the ortho position relative to the OH radical.

GROUP II:
- the OH radical is in the ortho position relative to the CHO function,
- the halogen atom X is in the para position relative to the OH radical,
- the radical $R_1$ is in the ortho position relative to the OH radical.

GROUP III:
- the radical OH is in the ortho position relative to the CHO function,
- the halogen atom X is in the ortho position relative to the OH radical,
- the radical $R_1$ is in the para position relative to the OH radical.

GROUP IV:
- the OH radical is in the meta position relative to the CHO function,
- the halogen atom X is in the ortho position relative to the OH radical,
- the radical $R_1$ is in the para position relative to the OH radical.

GROUP V:
- the OH radical is in the meta position relative to the CHO function,
- the halogen atom X is in the para position relative to the OH radical,

- the radical $R_1$ is in the ortho position relative to the OH radical.

5. Process according to any one of the claims 1 to 4, characterized in that the halogenobenzaldehyde complies with one of the following formulas:

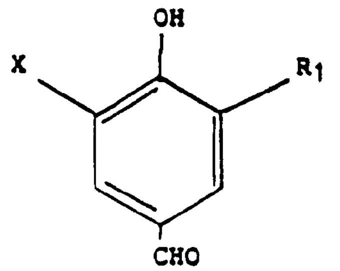

(Ia1)

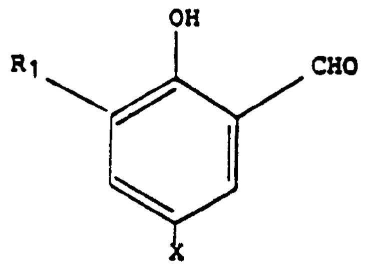

(Ia2)

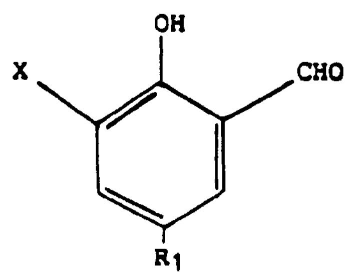

(Ia3)

in which
- X and $R_1$ have the meanings given hereinbefore.

6. Process according to any one of the claims 1 to 5, characterized in that the halogenobenzaldehyde is chosen from among:
   - 3-bromo-4-hydroxybenzaldehyde,
   - 3-iodo-4-hydroxybenzaldehyde,
   - 3,5-dibromo-4-hydroxybenzaldehyde
   - 3,5-diiodo-4-hydroxybenzaldehyde,
   - 3-bromo-5-methoxy-4-hydroxybenzaldehyde,
   - 3-iodo-5-methoxy-4-hydroxybenzaldehyde,
   - 3-bromo-5-ethoxy-4-hydroxybenzaldehyde,
   - 3-iodo-5-ethoxy-4-hydroxybenzaldehyde,
   - 3-bromo-4,5-dihydroxybenzaldehyde,
   - 3-iodo-4,5-dihydroxybenzaldehyde,
   - 3-bromo-2,5-dihydroxybenzaldehyde,
   - 3-iodo-2,5-dihydroxybenzaldehyde,
   - 2-bromo-4-hydroxybenzaldehyde,
   - 2-iodo-4-hydroxybenzaldehyde,
   - 4-bromo-3-hydroxybenzaldehyde,

- 4-iodo-3-hydroxybenzaldehyde,
- 3-bromo-2-hydroxybenzaldehyde,
- 3-iodo-2-hydroxybenzaldehyde,
- 5-bromo-2-hydroxybenzaldehyde,
- 5-iodo-2-hydroxybenzaldehyde.

**7.** Process according to any one of the claims 1 to 6, characterized in that the alkaline earth or alkali metal alkoxide complies with the general formula (II), in which R represents a straight or branched alkyl, alkenyl, alkadienyl or alkynyl radical preferably having less than 6 carbon atoms or a benzyl radical.

**8.** Process according to any one of the claims 1 to 7, characterized in that the alkali metal alkoxide is a sodium or potassium alkoxide of a primary or secondary alkanol having 1 to 4 carbon atoms.

**9.** Process according to any one of the claims 1 to 8, characterized in that the alkali metal alkoxide is sodium methylate or sodium ethylate.

**10.** Process according to any one of the claims 1 to 9, characterized in that working takes place in the presence of a carbonate of general formula (III):

$$[R_2\text{-O-CO-O-}]_n\text{-}R_3 \qquad \text{III}$$

in which:
- $R_2$ represents a straight or branched, alkyl radical having 1 to 6 carbon atoms, an aryl radical, an aryl radical substituted by one or two alkyl radicals having 1 to 12 carbon atoms, a cycloalkyl radical having 5 to 6 carbon atoms, a cycloalkyl radical having 5 or 6 carbon atoms substituted by 1 or 2 alkyl radicals having 1 to 12 carbon atoms, a $R_4$-O-CO- radical, in which $R_4$ represents a straight or branched, alkyl radical having 1 to 6 carbon atoms, an aryl radical, a cycloalkyl radical having 5 or 6 carbon atoms;
- $R_3$ represents a straight or branched, alkyl radical having 1 to 6 carbon atoms, an aryl radical, an aryl radical substituted by 1 or 2 alkyl radicals having 1 to 12 carbon atoms, a cycloalkyl radical having 5 or 6 carbon atoms, a cycloalkyl radical having 5 or 6 carbon atoms substituted by 1 or 2 alkyl radicals having 1 to 12 carbon atoms, an alkaline earth or alkali metal, preferably sodium or potassium;
- n=1 or n=2 when $R_3$ represents an alkaline earth metal;
- $R_2$ and $R_3$ together form an alkylene radical having 2 to 6 carbon atoms.

**11.** Process according to any one of the claims 1 to 10, characterized in that working takes place in the presence of a carbonate of general formula (IV):

$$R_2\text{-O-CO-O-}R_3 \qquad \text{(IV)}$$

in which,
- $R_2$ represents a straight or branched, alkyl radical having 1 to 6 carbon atoms, a phenyl radical, a phenyl radical substituted by 1 or 2 alkyl radicals having 1 to 4 carbon atoms, a cycloalkyl radical having 5 to 6 carbon atoms, a cycloalkyl radical having 5 or 6 carbon atoms substituted by 1 or 2 alkyl radicals having 1 to 4 carbon atoms, a $R_4$-O-CO- radical, in which $R_4$ represents a straight or branched, alkyl radical having 1 to 6 carbon atoms, a phenyl radical, a cycloalkyl radical having 5 or 6 carbon atoms;
- $R_3$ represents a straight or branched, alkyl radical having 1 to 6 carbon atoms, a phenyl radical, a phenyl radical substituted by 1 or 2 alkyl radicals having 1 to 4 carbon atoms, a cycloalkyl radical having 5 or 6 carbon atoms, a cycloalkyl radical having 5 or 6 carbon atoms substituted by 1 or 2 alkyl radicals having 1 to 4 carbon atoms, a sodium or potassium atom;
- $R_2$ and $R_3$ together form an alkylene radical having 2 to 6 carbon atoms.

**12.** Process according to any one of the claims 1 to 11, characterized in that use is made of carbon dioxide, dimethyl carbonate, diethyl carbonate, ethylene or propylene carbonate or ditert. butyl carbonate.

**13.** Process according to any one of the claims 1 to 12, characterized in that the copper catalyst is metallic copper or a compound of copper, which is an organic or inorganic compound of copper I or copper II, chosen from among cuprous chloride, cupric chloride, cuprous carbonate, basic copper II carbonate, cuprous nitrate, cupric nitrate, cupric sulphate, cupric acetate, cupric trifluoromethyl sulphonate, cupric hydroxide, copper II picolinate, copper I methylate, copper II methylate, 8-quinoline-copper II chelate and compounds of copper of formula $ClCuOCH_3$, $Cu_2(OCH_3)_2(acac)_2$.

14. Process according to any one of the claims 1 to 13, characterized in that the alkaline earth or alkali metal alkoxide quantity used is equal to or greater than the stoichiometric quantity necessary for transforming the compound of formula (I) into the corresponding alkaline earth or alkali metal phenoxide and for transforming its halogen atom or atoms into alkoxy groups.

15. Process according to any one of the claims 1 to 14, characterized in that the molar ratio of the copper catalyst to the compound of formula (I) is 1 to 50% and preferably 2 to 20%.

16. Process according to any one of the claims 1 to 15, characterized in that the reaction is performed in a solvent constituted by the alk nol corresponding to the alkaline earth or alkali metal alkoxide used.

17. Process according to claim 16, characterized in that the initial concentration of the compound of formula (I) relative to the mixture of the compound of formula (I) and the solvent is 3 to 40 % by weight and preferably 10 to 30 % by weight.

18. Process according to any one of the claims 1 to 17, characterized in that the molar ratio of the carbonate of formula (III) or (IV) to the copper catalyst is 1 to 50 and preferably 1 to 10.

19. Process according to any one of the claims 1 to 18, characterized in that working takes place at a temperature of 90 to 220°C and preferably 100 to 180°C.

20. Application of the process described in any one of the claims 1 to 19 to the preparation of 3,4,5-trimethoxybenzaldehyde, 4-hydroxy-3-methoxybenzaldehyde and 2-hydroxy-5-methoxybenzaldehyde.

## Patentansprüche

1. Verfahren zur Herstellung von Alkoxybenzaldehyden durch Reaktion :
   - eines Halogenbenzaldehyds der allgemeinen Formel (I):

$$X - R_o - CHO \qquad (I)$$

   in der:
   - X ein Halogen-, bevorzugt ein Iod-, Brom- oder Chloratom bedeutet,
   - $R_o$ einen mindestens fünfgliedrigen cycloaromatischen eventuell substituierten Cyclus bedeutet und für mindestens einen der folgenden Substituenten steht:
     . einem mono- oder polycyclischen carbocyclischen aromatischen Rest,
     . einem mono- oder polycyclischen heterocyclischen aromatischen Rest mit mindestens einem der Heteroatome O, N und S,
   - und eines Alkalimetallakoholats der allgemeinen Formel (II) :

$$M^{m+} [ O - R ]_m^- \qquad (II)$$

   in der :
   - M ein Alkali- oder Erdalkalimetall bedeutet,
   - m die Bindigkeit des Alkali- oder Erdalkalimetalls bedeutet,
   - R eine Kohlenwasserstoffgruppe mit 1 bis 12 Kohlenstoffatomen bedeutet, die eine gesättigte oder ungesättigte acyclische verzweigte oder unverzweigte aliphatische oder arylaliphatische Gruppe sein kann,

   in Anwesenheit von Kupfer und/oder einer Kupferverbindung, dadurch gekennzeichnet, daß in einer ausreichenden Menge eines organischen Carbonates, eines organometallischen gemischten Carbonates, von Kohlendioxid oder einer zur Bildung von Kohlendioxid im Reaktionsmilieu geeigneten Verbindung gearbeitet wird.

2. Verfahren nach Anspruch 1, dadurch gekennzeichnet, daß das Halogenbenzaldehyd der allgemeinen Formel (I) entspricht, worin der Rest $R_o$ bedeutet :
   1° - eine mono- oder polycyclische aromatische carbocyclische Gruppe.
   2° - eine mono- oder polycyclische aromatische heterocylische Gruppe.
   3° - eine zweibindige Gruppe, gebildet durch Verkettung von Gruppierungen, wie unter den Punkten 1 und/oder 2 definiert, die untereinander verbunden sind über:
   - eine Valenzbindung,
   - eine Alkylen- oder Alkylidengruppe mit 1 bis 4 Kohlenstoffatomen, bevorzugt eine Methylen- oder Isopropylidengruppe, eine der folgenden Gruppen:

$-O-$ , $-CO-$ , $-COO-$ ,

$-S-$ , $-SO-$ , $-SO_2-$ ,

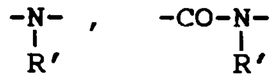

wobei in diesen Formeln R' eine Alkylgruppe mit 1 bis 4 Kohlenstoffatomen, ein Cyclohexyl- oder eine Phenylgruppe bedeutet.

3. Verfahren nach einem der Ansprüche 1 und 2, dadurch gekennzeichnet, daß das Halogenbenzaldehyd der allgemeinen Formel (Ia) entspricht :

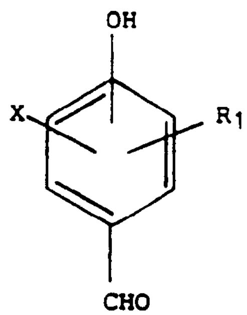

(Ia)

in der :

- X ein Jod-, Brom- oder Chloratom bedeutet,
- $R_1$ ein Wasserstoff-, ein Jod-, Brom-, ein Chloratom, eine Alkylgruppe mit 1 bis 4 Kohlenstoffatomen, eine Alkyloxygruppe mit 1 bis 4 Kohlenstoffatomen, eine Hydroxygruppe bedeutet, in der genannten Formel (Ia) kann die Hydroxygruppe ortho-, meta- oder paraständig zur Aldehydfunktion sein.

4. Verfahren nach einem der Ansprüche 1 bis 3, dadurch gekennzeichnet, daß das Halogenbenzaldehyd der allgemeinen Formel (Ia) entspricht, in der :

. Gruppe I :
  - die Hydroxygruppe paraständig zur CHO-Funktion ist,
  - das Halogenatom X orthoständig zur OH-Funktion ist,
  - die $R_1$-Gruppe orthoständig zur OH-Funktion ist.

. Gruppe II :
  - die Hydroxygruppe orthoständig zur CHO-Funktion ist,
  - das Halogenatom X paraständig zur OH-Funktion ist,
  - die $R_1$-Gruppe orthoständig zur OH-Funktion ist.

. Gruppe III :
  - die Hydroxygruppe orthoständig zur CHO-Funktion ist,
  - das Halogenatom X orthoständig zur OH-Funktion ist,
  - die $R_1$-Gruppe paraständig zur OH-Funktion ist.

. Gruppe IV :
  - die Hydroxygruppe metaständig zur CHO-Funktion ist,
  - das Halogenatom X orthoständig zur OH-Funktion ist,
  - die $R_1$-Gruppe paraständig zur OH-Funktion ist.

. Gruppe V :
  - die Hydroxygruppe metaständig zur CHO-Funktion ist,
  - das Halogenatom X paraständig zur OH-Funktion ist,
  - die $R_1$-Gruppe orthoständig zur OH-Funktion ist.

5. Verfahren nach einem der Ansprüche 1 bis 4, dadurch gekennzeichnet, daß das Halogenbenzaldehyd einer der folgenden Formeln entspricht :

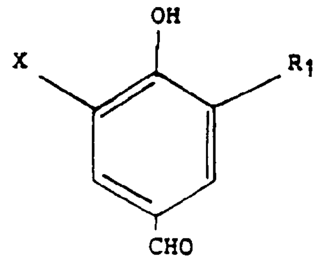

(Ia1)

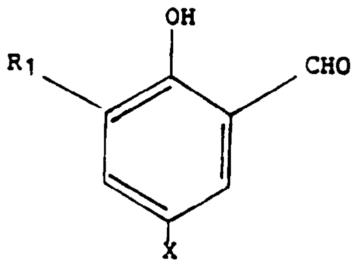

(Ia2)

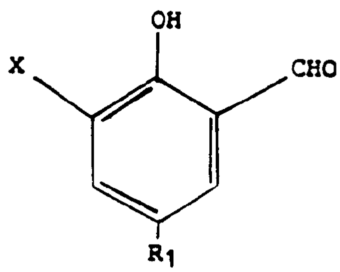

(Ia3)

in denen :

- X und $R_1$ die vorhergehend zugeordneten Bedeutungen tragen.

**6.** Verfahren nach einem der Ansprüche 1 bis 5, dadurch gekennzeichnet, daß das Halogenbenzaldehyd ausgewählt wird unter :

- 3-Brom-4-hydroxybenzaldehyd
- 4-Hydroxy-3-jodbenzaldehyd
- 3,5-Dibrom-4-hydroxybenzaldehyd
- 4-Hydroxy-3,5-dijodbenzaldehyd
- 3-Brom-4-hydroxy-5-methoxybenzaldehyd
- 4-Hydroxy-3-jod-5-methoxybenzaldehyd
- 3-Brom-5-ethoxy-4-hydroxybenzaldehyd
- 5-Ethoxy-4-hydroxy-3-jodbenzaldehyd
- 3-Brom-4,5-dihydroxybenzaldehyd
- 4,5-Dihydroxy-3-jodbenzaldehyd
- 3-Brom-2,5-hydroxybenzaldehyd
- 2,5-Dihydroxy-3-jodbenzaldehyd
- 2-Brom-4-hydroxybenzaldehyd
- 4-Hydroxy-2-jodbenzaldehyd
- 4-Brom-3 -hydroxybenzaldehyd
- 3-Hydroxy-4- jodbenzaldehyd
- 3-Brom-2-hydroxybenzaldehyd
- 2-Hydroxy-3-jodbenzaldehyd
- 5-Brom-2-hydroxybenzaldehyd
- 2-Hydroxy-5-jodbenzaldehyd

7. Verfahren nach einem der Ansprüche 1 bis 6, dadurch gekennzeichnet, daß das Alkali- oder Erdalkalimetallalkoholat der allgemeinen Formel (II) entspricht, in der R eine verzweigte oder unverzweigte Alkyl-, eine Alkenyl-, eine Alkandienyl-, Alkinylgruppe, mit bevorzugt weniger als 6 Kohlenstoffatomen, oder eine Benzylgruppe bedeutet.

8. Verfahren nach einem der Ansprüche 1 bis 7, dadurch gekennzeichnet, daß das Alkalimetallalkoholat ein Natrium- oder Kaliumalkoholat eines primären oder sekundären Alkanols mit 1 bis 4 Kohlenstoffatomen ist.

9. Verfahren nach einem der Ansprüche 1 bis 8, dadurch gekennzeichnet, daß das Alkalimetallalkoholat ein Natrium- methanolat oder Natriumethanolat ist.

10. Verfahren nach einem der Ansprüche 1 bis 9, dadurch gekennzeichnet, daß in Gegenwart eines Carbonats der allgemeinen Formel (III) gearbeitet wird :

$$[R_2\text{-O-CO-O-}]_n\text{-}R_3 \qquad \text{(III)}$$

hierin bedeuten :

- $R_2$
  - eine verzweigte oder unverzweigte Alkylgruppe mit 1 bis 6 Kohlenstoffatomen ;
  - eine Arylgruppe ;
  - eine durch 1 oder 2 Alkylgruppen, mit 1 bis 12 Kohlenstoffatomen, substituierte Arylgruppe ;
  - eine Cycloalkylgruppe mit 5 bis 6 Kohlenstoffatomen ;
  - eine Cycloalkylgruppe mit 5 bis 6 Kohlenstoffatomen, die mit einer oder zwei Alkylgruppen, mit 1 bis 12 Kohlenstoffatomen, substituiert ist ;
  - eine $R_4$-O-CO-Gruppe, in der $R_4$ eine verzweigte oder unverzweigte Alkylgruppe mit 1 bis 6 Kohlenstoffatomen, eine Arylgruppe, eine Cycloalkylgruppe mit 5 bis 6 Kohlenstoffatomen ;
- $R_3$
  - eine verzweigte oder unverzweigte Alkylgruppe mit 1 bis 6 Kohlenstoffatomen ;
  - eine Arylgruppe ;
  - eine durch 1 oder 2 Alkylgruppen mit 1 bis 12 Kohlenstoffatomen substituierte Arylgruppe ;
  - eine Cycloalkylgruppe mit 5 oder 6 Kohlenstoffatomen ;
  - eine Cycloalkylgruppe mit 5 oder 6 Kohlenstoffatomen, die mit einer oder zwei Alkylgruppen mit 1 bis 12 Kohlenstoffatomen substituiert ist ;
  - ein Alkali- oder Erdalkalimetall, bevorzugt Natrium oder Kalium ;
- n = 1 oder, wenn $R_3$ ein Erdalkalimetall bedeutet, n = 2 ;
- $R_2$ und $R_3$ bilden gemeinsam eine Alkylengruppe mit 2 bis 6 Kohlenstoffatomen.

11. Verfahren nach einem der Ansprüche 1 bis 10, dadurch gekennzeichnet, daß in Gegenwart eines Carbonates der allgemeinen Formel (IV) gearbeitet wird:

$$R_2\text{-O-CO-O } R_3 \qquad \text{(IV)}$$

hierin bedeuten :

$R_2$

- eine verzweigte oder unverzweigte Alkylgruppe mit 1 bis 6 Kohlenstoffatomen ;
- eine Phenylgruppe ;
- eine durch 1 oder 2 Alkylgruppen, mit 1 bis 4 Kohlenstoffatomen, substituierte Phenylgruppe ;
- eine Cycloalkylgruppe mit 5 oder 6 Kohlenstoffatomen ;
- eine Cycloalkylgruppe mit 5 oder 6 Kohlenstoffatomen, die mit einer oder zwei Alkylgruppen, mit 1 bis 4 Kohlenstoffatomen, substituiert ist ;
- eine $R_4$-O-CO-Gruppe, in der $R_4$ eine verzweigte oder unverzweigte Alkylgruppe mit 1 bis 6 Kohlenstoffatomen, eine Phenylgruppe, eine Cycloalkylgruppe mit 5 bis 6 Kohlenstoffatomen bedeutet ;
- $R_3$
  - eine verzweigte oder unverzweigte Alkylgruppe mit 1 bis 6 Kohlenstoffatomen ;
  - eine Phenylgruppe ;
  - eine durch 1 oder 2 Alkylgruppen, mit 1 bis 4 Kohlenstoffatomen, substituierte Phenylgruppe ;
  - eine Cycloalkylgruppe mit 5 oder 6 Kohlenstoffatomen ;
  - eine Cycloalkylgruppe mit 5 oder 6 Kohlenstoffatomen, die mit einer oder zwei Alkylgruppen, mit 1 bis 4 Kohlenstoffatomen, substituiert ist ;
  - ein Natrium- oder Kaliumatom ;
- $R_2$ und $R_3$ bilden gemeinsam eine Alkylengruppe mit 2 bis 6 Kohlenstoffatomen.

12. Verfahren nach einem der Ansprüche 1 bis 11, dadurch gekennzeichnet, daß Kohlendioxid, Dimethylcarbonat, Diethylcarbonat, Ethylen-, Propylencarbonat, Ditertbutyldicarbonat eingesetzt werden.

13. Verfahren nach einem der Ansprüche 1 bis 12, dadurch gekennzeichnet, daß der Kupferkatalysator aus Kupfermetall oder einer Kupferverbindung besteht, die eine organische oder anorganische Kupfer-(I)- oder Kupfer-(II)-verbindung ist, ausgewählt aus Kupfer-(I)-chlorid, Kupfer-(II)-chlorid, Kupfer-(I)-carbonat, basischem Kupfer-(II)-carbonat, Kupfer-(I)-nitrat, Kupfer-(II)-nitrat, Kupfer-(II)-sulfat, Kupfer-(II)-acetat, Kupfer-(II)-trifluormethylsulfonat, Kupfer-(II)-hydroxyd, Kupfer-(II)- picolinat, Kupfer-(I)-methanolat, Kupfer-(II)-methanolat, dem Chelatkomplex 8-Chinoleinkupfer(II), den Kupferverbindungen der Formel $ClCuOCH_3$, $Cu_2(OCH_3)_2$ $(acac)_2$.

14. Verfahren nach einem der Ansprüche 1 bis 13, dadurch gekennzeichnet, daß die eingesetzte Erdalkali- oder Alkalialkoholatmenge gleich oder größer als die stöchiometrisch geforderte Menge zur Umwandlung der Verbindung der Formel (I) in das entsprechende Erdalkali-oder Alkaliphenolat und zur Umwandlung des oder der Halogenatome, die sie besitzt, in Alkoxygruppen ist.

15. Verfahren nach einem der Ansprüche 1 bis 14, dadurch gekennzeichnet, daß das Molverhältnis Kupferkatalysator/Verbindung der Formel (I) 1 % bis 50 % und bevorzugt 2 % bis 20 % beträgt.

16. Verfahren nach einem der Ansprüche 1 bis 15, dadurch gekennzeichnet, daß die Reaktion in einem Alkanol als Lösungsmittel durchgeführt wird, der dem eingesetzten Erdalkali- oder Alkalialkoholat entspricht.

17. Verfahren nach Anspruch 16, dadurch gekennzeichnet, daß die Anfangskonzentration der Verbindung mit Formel (I), die Mischung Verbindung mit Formel (I)/Lösungsmittel betreffend, 3 bis 40 Gewichtsprozente und bevorzugt 10 % bis 30 % beträgt.

18. Verfahren nach einem der Ansprüche 1 bis 17, dadurch gekennzeichnet, daß das Molverhältnis Carbonat der Formel (II) oder (IV)/Kupferkatalysator 1 bis 50 und bevorzugt 1 bis 10 beträgt.

19. Verfahren nach einem der Ansprüche 1 bis 18, dadurch gekennzeichnet, daß bei einer Temperatur von 90°C bis 220°C und bevorzugt von 100°C bis 180°C gearbeitet wird.

20. Anwendung des in einem der Ansprüche 1 bis 19 beschriebenen Verfahrens zur Herstellung von 3,4,5-Trimethoxybenzaldehyd, 4-Hydroxy-3-methoxybenzaldehyd und 2-Hydroxy-5-methoxybenzaldehyd.